(19)

Europäisches
Patentamt
European
Patent Office
Office européen
des brevets

(11) **EP 2 447 866 A1**

(12) **EUROPEAN PATENT APPLICATION**

(43) Date of publication:
**02.05.2012 Bulletin 2012/18**

(51) Int Cl.:
**G06F 19/00** (2011.01)

(21) Application number: **10189084.6**

(22) Date of filing: **27.10.2010**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(71) Applicant: **Koninklijke Philips Electronics N.V.**
**5621 BA Eindhoven (NL)**

(72) Inventors:
• **Barroso, Andre, M.**
**5600 AE, Eindhoven (NL)**

• **Sarroukh, Bahaa, E.**
**5600 AE, Eindhoven (NL)**
• **Schlangen, Lucas, J., M.**
**5600 AE, Eindhoven (NL)**

(74) Representative: **Damen, Daniel Martijn**
**Philips**
**Intellectual Property & Standards**
**P.O. Box 220**
**5600 AE Eindhoven (NL)**

(54) **Method for determining a feature of the circadian rhythm of a subject**

(57) The invention relates to method for determining a feature of the circadian rhythm of the subject, comprising measuring a first input signal indicating a cardiac function of the subject, measuring at least one second input signal indicating the activity of the subject, combining the first input signal and the second input signal to a periodic output signal representing the circadian rhythm of the subject, and determining at least one feature of this periodic output signal.

**Description**

FIELD OF THE INVENTION

The invention relates to a method of determining a feature of the circadian rhythm of a subject.

BACKGROUND OF THE INVENTION

The presence of day-night variations in cardiovascular and metabolic function is well known. However, only recently it has been shown that cardiovascular and metabolic processes are not only affected by the behavioral sleep/wake cycle, but is partly under direct control of a master circadian pacemaker. This relation between the master circadian pacemaker and cardiac output suggests that the latter can be used to reliably derive oscillation parameters of the former such as amplitude and acrophase, i. e., the time of the oscillation peak. These parameters have important applications in improving human sleep and physical or mental performance.

Knowing the circadian phase of an individual helps to optimize the timing of light interventions that allow shifting the circadian rhythm. Such a shifting of circadian rhythms is of use for treating jet-lag, helping shift workers adapt to night shift works schedules and to guide peoples with sleep disorders like delayed sleep phase syndrome or advanced sleep phase syndrome to more normal timing of their sleep-wake patterns. Optimizing the circadian phase of an individual allows adjusting the biorhythm of an individual so that maximum alertness and performance occur at the desired timing. Daily living, job performance, sport performance, etc. can be expected to benefit from this.

A major advantage of using cardiac output as a means to estimate the dynamics of the master circadian pacemaker is that it can be reliably measured using relatively unobstrusive sensors. The data these sensors generate can be used to collect signals that contain small endogenous circadian variations. Given the small amplitude of these variations, these are often masked by the influence of competing processes, e. g. the impact of physical or mental stress on heart activity. Many of these masking effects produce disturbances that are easy to filter as they introduce frequency components outside the normal circadian range. However, other masking effects are also circadian in nature and require more elaborate filtering methods. Recent studies suggested that the circadian modulation of the cardiac activity is influenced by prior wakefulness periods. The sleep/wake cycle follows a circadian cycle that affect cardiac activity in a similar frequency range than the master circadian pacemaker.

SUMMARY OF THE INVENTION

It is therefore an object of the present invention to provide a method for determining a feature of the circadian rhythm of a subject that allows an estimation of the master pacemaker oscillation parameters by analyzing the cardiac function of a subject, removing the masking effects of the endogenous circadian rhythm present in the cardiac function.

This object is achieved by a method comprising the features of claim 1.

According to this method, a first input signal is measured that is related to a cardiac function of the subject over a certain time period. Additionally a second input signal is measured that indicates the physiological activity of the subject over a time period overlapping this first time period in which the first input signal is measured.

The first input signal and the second input signal are combined to result in a periodic output signal representing the circadian rhythm of the subject. From this output signal, the desired feature of the circadian rhythm is derived.

The idea behind the combination of a first input signal and the second input signal is that the latter includes information on effects that mask the information about the endogenous circadian rhythm that is present in the first signal. The combination allows to remove the unwanted features from the first input signal (representing the cardiac function) so that oscillation parameters describing the dynamics of the circadian rhythm can directly be derived from the periodic output signal.

The combination of the first input signal and the second input signal can be performed by autoregression, decoloration, independent component analysis or (nonlinear) principle component analysis, leading to an enhanced representation of circadian rhythm by the periodic output signal. This enhanced signal can then be used to derive an estimation of the oscillation parameters, for instance by applying harmonic regression.

While the first input signal can be any marker of a cardiac function, the second signal can be represented by physiological, behavioral or environmental data from the subject that provide an indication of her/his prior period of wakefulness, e. g. wrist actigraphy, sleep locks, questionnaire answers, skin temperature, light exposure, etc. over a period of time that overlaps with the time period over which the cardiac function is measured.

Preferably the feature of the circadian rhythm to be determined is the acrophase of the periodic output signal.

According to another preferred embodiment of the method according to the present invention, said feature is the amplitude of the periodic output signal.

In another preferred embodiment, the first input signal and the second input signal are gained by sampling to result in a number of first input signal values and second input signal values, respectively, and a number of output signal values is computed from the sampled first input signal values and a second input signal values.

In this embodiment the first and second input signals are discrete input signal values gained by sampling a cardiac function and monitoring the activity of the subject, respectively. This results in a first set of first input signal values and a second set of second input signal values. From these sets, a set of output signal values can be derived, for example, by solving a system of equations that includes the first and second input signal values as input variables, to get the output signal values as solutions.

Preferably the periodic output signal is derived from the computed number of output signal values by regression analysis.

According to another preferred embodiment of the present invention, the number of output signal values is computed from the first input signal values and second input signal values according to an autoregressive moving average model (ARMAX) with the first input signal values and second input signal values as exogenous inputs.

Autoregressive moving average models are commonly known in signal processing to process autocorrelated time series data. The model consists of two parts, namely an autoregressive part and a moving average part. Its mathematical basis is a linear equation system to set two time series into a relation to each other, here represented by the first input signal values and the second input signal values. Solving this linear equation system results in a series of output signal values representing the circadian rhythm of the subj ect.

Preferably the first input signal values and the second input signal values are gained by sampling at a frequency above 1 Hz.

According to another preferred embodiment, the first input signal values or the second input signal values are gained by sampling original signal values at a first frequency, said original signal values being gained by sampling at a second frequency higher than the first frequency.

In practice this means that one of the time series, represented by the first input signal values or the second input values, is gained by sampling original (raw) signal values that are, in turn, gained by sampling a measurement parameter at a relatively high frequency. Just to give one example, the original signal values are raw values gained by sampling a cardiac parameter at a frequency of 512 Hz. These original signal values cannot be computed directly together with a second time series of second input signal values that are gained by sampling at 60 Hz. Therefore the original signal values are sampled at a relatively low frequency of 60 Hz to gain the first input signal values, which can then be combined with a second input signal values, for example, by the ARMAX model mentioned above.

Preferably the first input signal is the heart beat interval (IBI) of the subject.

According to another preferred embodiment, raw ECG signal values are gained by sampling a raw ECG signal at a second frequency of 512 Hz, an IBI signal is derived from the gained ECG signal values, and IBI signal values representing the first input signal values are gained by sampling the derived IBI signal at a first frequency of 60 Hz.

In another preferred embodiment of the present invention, the second input signal is a wrist actigraphy signal of the subject.

According to still another preferred embodiment, the second input signal is a signal indicating the light exposure of the subject.

BRIEF DESCRIPTION OF THE DRAWINGS

These and other aspects of the invention will be apparent from and elucidated with reference to the embodiments described hereinafter.

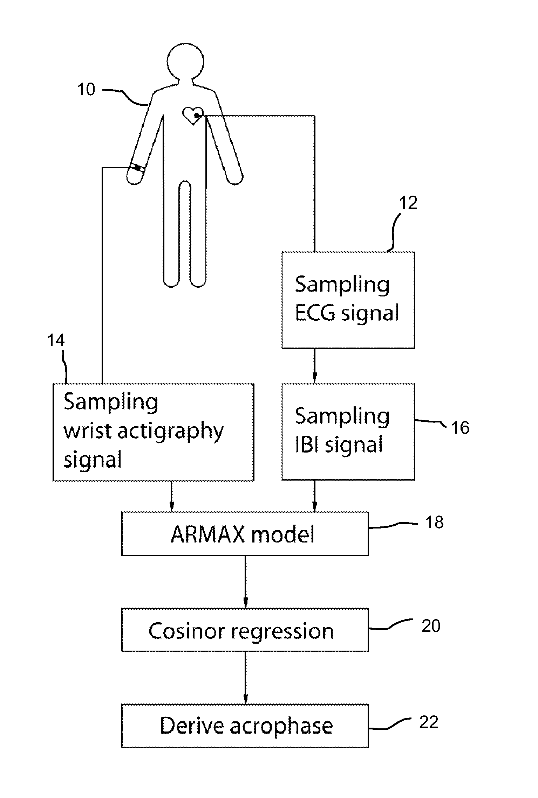

In the drawings, the only figure is a schematic flow diagram to illustrate one embodiment of the method for determining a feature of the circadian rhythm of a subject according to the present invention.

DETAILED DESCRIPTION OF EMBODIMENTS

In the method shown in the flow diagram of the figure, physiological signals of a subject 10 are collected. ECG signals are gained by means of a suitable sensor integrated in a wearable ECG device in the form of a strap belt or the like. Raw ECG signal values are gained by sampling at a frequency of 512 Hz (step 12). This sampling is performed over a predetermined time period T to result in a set of raw ECG signal values. Secondly, additional physiological data are collected from the subject 10 by means of another suitable sensor. These second data indicate the physiological activity of the subject 10 over a time period overlapping the time period T mentioned before for sampling the raw ECG signal. This sampling of an activity related signal is performed at a lower frequency of 60 Hz (step 14). A suitable sensor can be integrated in a wrist band or the like to measure a wrist activity signal of the subject 10. It is noted that these second physiological data that indicate the activity of the subject 10 can also be other data based on sleep logs, skin temperature, light exposure, etc. It is assumed that these data contain information on a prior wakefulness period of the subject 10. In the following it will be shown that these data can be combined with data related to the cardiac function of the subject 10 to remove masking effects from the cardiac function signals, resulting in a signal function representing the circadian activity of the subject 10.

From the raw ECG signals, an IBI signal is derived by computing R-R intervals. The resulting IBI signal is further sampled in step 16 at a frequency of 60 Hz. The result of step 16 is a set of first input signal values related to the IBI of the subject 10 in the time interval T. The result of step 14, on the other hand, is a set with the same number of second input signal values indicating the activity of the subject 10 in a time period overlapping the time period T. The first and second input signals are detrended to eliminate zero frequency components.

In the following step 18, the first input signal values and the second input signal values are mathematically combined with the help of an autoregressive moving average model (ARMAX) in which the first input signal values and the second input signal values represent exogenous inputs. Let $x_n$ be the second input signal values and $x'_n$ the first input signal values, it is possible within the ARMAX model to calculate a series of $y_n$ output signal values representing a linear function of $x_n$ and $x'_n$. This function $y_n$ is an enhanced circadian rhythm representation containing an information about the acrophase of the circadian rhythm. $y_n$ for any n can be computed for $x_n$, $x'_n$ through a linear combination of past values.

Described in more detail, the first and second output signal values $x_n$ and $x'_n$ can be combined for obtaining an enhanced circadian rhythm representation $y_n$ via the following linear model:

$$A(q)y_n = B(q)\begin{bmatrix} x_n \\ x'_n \end{bmatrix} + C(q)w_n \tag{1}$$

when n is an non-negative integer, q is a backward shift operator, i. e. $qy_n = y_{n-1}$, $w_n$ is a Gaussian white noise process with mean 0 and standard deviation σ and A (q), B (q) and C (q) are polynominals defined as follows:

$$A(q) = 1 + a_1 q + a_2 q^2 + ... + a_r q^r \tag{2}$$

$$B(q) = \begin{bmatrix} b_{11}q + b_{12}q^2 + ... + b_{1s_1}q^{s_1} \\ b_{21}q + b_{22}q^2 + ... + b_{2s_2}q^{s_2} \end{bmatrix}^T \tag{3}$$

$$C(q) = 1 + c_1 q + c_2 q^2 + ... + c_u q^u \tag{4}$$

In these expressions, the values r, $[s_1 \ s_2]^T$, u $a_i$, $b_{ij}$, $c_i$ and σ are given constants of the model. The enhanced rhythm representation $y_n$ is then subject to a cosinor regression (step 20) of the form:

$$\mu + \alpha \cos(2\pi n \Delta t + \Phi) \tag{5}$$

The regression of coefficient φ is the desired estimator of the circadian rhythm. φ represents the acrophase, i. e. the time of oscillation peak of the circadian rhythm function that can be derived from the structure of equation (5) above (step 22). Once the coefficient φ is derived, it can be used to control the timing of a lighting intervention that allows a shifting of the circadian rhythm of the subject 10 and to help to optimize the timing of the circadian activity. For example, more close to the time at which the core body temperature is minimum (around two hours before spontaneous wake up on a free day), light interventions become more effective and shorter treatment durations can be used.

It is noted that the coefficient $\phi$, representing the acrophase of the circadian rhythm function, is only one example of a feature of the circadian rhythm represented by the periodic output signal $x_n$. For example, the amplitude of this signal might be another feature that can be used to control a lighting intervention to optimize the circadian phase of the subject 10. Generally another cardiac function than the heartbeat interval (IBI) of the subject 10 can be used as a first input signal, while another second input signal different from a wrist actigraphy signal can be used to represent the activity of the subject 10, as already mentioned above.

While the invention has been illustrated and described in detail in the drawings and foregoing description, such illustration and description are to be considered illustrative or exemplary and not restrictive; the invention is not limited to the disclosed embodiments. Other variations to the disclosed embodiments can be understood and effected by those skilled in the art in practicing the claimed invention, from a study of the drawings, the disclosure, and the appended claims. In the claims, the word "comprising" does not exclude other elements or steps, and the indefinite article "a" or "an" does not exclude a plurality. The mere fact that certain measures are recited in mutually different dependent claims does not indicate that a combination of these measures cannot be used to advantage. Any reference signs in the claims should not be construed as limiting the scope.

## Claims

1. A method for determining a feature of the circadian rhythm of a subject (10), comprising the following steps:

   - measuring a first input signal $x'_n$ indicating a cardiac function of the subject (10) over a time period T;
   - measuring at least one second input signal $x_n$ indicating the activity of the subject (10) over a time period overlapping the time period T;
   - combining the first input signal $x'_n$ and the second input signal $x_n$ to a periodic output signal $y_n$ representing the circadian rhythm of the subject, and
   - determining at least one feature of this periodic output signal $y_n$.

2. The method according to claim 1,
   wherein said feature is the acrophase of the periodic output signal $y_n$.

3. The method according to claim 1 or 2,
   wherein said feature is the amplitude of the periodic output signal $y_n$.

4. The method according to claim 1 to 3,
   wherein the first input signal and the second input signal are gained by sampling to result in a number of first input signal values $x'_n$ and second input signal values $x_n$, respectively,
   and a number of output signal values $y_n$ is computed from the sampled first input signal values $x'_n$ and second input signal values $x_n$,.

5. The method according to claim 4,
   wherein the periodic output signal is derived from the computed number of output signal values $y_n$ by regression analysis.

6. The method according to claim 4 or 5,
   wherein the number of output signal values $y_n$ is computed from the first input signal values $x'_n$ and second input signal values $x_n$ according to an autoregressive moving average model (ARMAX) with the first input signal values $x'_n$ and second input signal values $_{Xn}$ as exogenous inputs.

7. The method according to one of claims 4 to 6,
   wherein the first input signal values $x'_n$ and the second input signal values $x_n$ are gained by sampling at a frequency above 1 Hz.

8. The method according to one of claims 4 to 7,
   wherein the first input signal values $x'_n$ or the second input signal values $x_n$ are gained by sampling original signal values at a first frequency,
   said original signal values being gained by sampling at a second frequency higher than the first frequency.

9. The method according to one of the preceding claims,
   wherein the first input signal is the heart beat interval (IBI) of the subject (10).

10. The method according to claim 9 in connection with claim 8,
    wherein raw ECG signal values are gained by sampling a raw ECG signal at a second frequency of 512 Hz,
    an IBI signal is derived from the gained ECG signal values,
    and IBI signal values representing the first input signal values are gained by sampling the derived IBI signal at a first frequency of 60 Hz.

11. The method according to one of the preceding claims,
    wherein the second input signal is a wrist actigraphy signal of the subject (10).

12. The method according to one of claims 1 to 10,
    wherein the second input signal is a signal indicating the light exposure of the subject (10).

Europäisches
Patentamt

European
Patent Office

Office européen
des brevets

**EUROPEAN SEARCH REPORT**

Application Number

EP 10 18 9084

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | PLAMEN. CH. IVANOV: "Scale-invariant Aspects of Cardiac Dynamics Across Sleep Stages and Circadian Phases", PROCEEDINGS OF THE 28TH IEEE EMBS ANNUAL INT. CONF., 30 August 2006 (2006-08-30), - 3 September 2006 (2006-09-03), pages 445-448, XP002630659, * Chapters I. and II. * ----- | 1-12 | INV. G06F19/00 |
| Y | WO 98/46128 A1 (HEARTLINK PTY LTD [AU]; STAMFER HANS GEORGE [AU]) 22 October 1998 (1998-10-22) * page 4, line 17 - page 5, line 26 * ----- | 1-12 | |
| Y | WO 2007/143535 A2 (BIANCAMED LTD [IE]; HENEGHAN CONOR [IE]; HANLEY CONOR [IE]; FOX NIALL) 13 December 2007 (2007-12-13) * figure 1 * * paragraph [0080] - paragraph [0087] * ----- | 1-12 | |
| A | US 6 128 534 A (PARK EULJOON [US] ET AL) 3 October 2000 (2000-10-03) * column 4, line 40 - column 9, line 53 * ----- | 1 | TECHNICAL FIELDS SEARCHED (IPC) G06F A61B |
| A | A. WIRZ-JUSTICE: "How to measure circadian rhythms in humans", MEDICOGRAPHIA, vol. 29, no. 1, 2007, pages 84-90, XP002630660, * the whole document * ----- | 1 | |

The present search report has been drawn up for all claims

| Place of search | Date of completion of the search | Examiner |
|---|---|---|
| Munich | 31 March 2011 | Rinelli, Pietro |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 10 18 9084

This annex lists the patent family members relating to the patent documents cited in the above-mentioned European search report.
The members are as contained in the European Patent Office EDP file on
The European Patent Office is in no way liable for these particulars which are merely given for the purpose of information.

31-03-2011

| Patent document cited in search report | | Publication date | Patent family member(s) | | Publication date |
|---|---|---|---|---|---|
| WO 9846128 | A1 | 22-10-1998 | BR | 9808446 A | 23-05-2000 |
| | | | CA | 2284553 A1 | 22-10-1998 |
| | | | CN | 1251974 A | 03-05-2000 |
| | | | EP | 1014851 A1 | 05-07-2000 |
| | | | IL | 132186 A | 20-03-2008 |
| | | | JP | 2001518823 T | 16-10-2001 |
| | | | NO | 994689 A | 13-12-1999 |
| | | | NZ | 337833 A | 29-06-2001 |
| | | | PL | 336149 A1 | 05-06-2000 |
| | | | US | 6245021 B1 | 12-06-2001 |
| WO 2007143535 | A2 | 13-12-2007 | AU | 2007256872 A1 | 13-12-2007 |
| | | | CA | 2654095 A1 | 13-12-2007 |
| | | | CN | 101489478 A | 22-07-2009 |
| | | | EP | 2020919 A2 | 11-02-2009 |
| | | | JP | 2009538720 T | 12-11-2009 |
| | | | US | 2009203972 A1 | 13-08-2009 |
| US 6128534 | A | 03-10-2000 | NONE | | |

EPO FORM P0459

For more details about this annex : see Official Journal of the European Patent Office, No. 12/82